# EUROPEAN PATENT SPECIFICATION

(11) **EP 1 435 991 B1**
(45) Date of publication and mention of the grant of the patent: **15.10.2008**
(21) Application number: 02770106.9
(22) Date of filing: 21.10.2002
(51) Int. Cl.: A61K 38/15, A61P 35/00

(54) **USE OF APLIDINE FOR THE TREATMENT OF PANCREATIC CANCER**
VERWENDUNG VON APLIDINE IN DER BEHANDLUNG VON PANKREASKREBS
UTILISATION D'APLIDINE POUR LE TRAITEMENT DU CANCER DU PANCREAS

(30) Priority: 19.10.2001 US 348412 P; 15.04.2002 GB 0208624
(43) Date of publication of application: 14.07.2004
(73) Proprietor: PHARMA MAR, S.A., 28760 Tres Cantos, Madrid (ES)
(72) Inventor: MANGUES, R., I. Ric. del H. - Santa Cruz/Pablo, 08025 Barcelona (ES); HENRIQUEZ, R., NeuroPharma S.A.U., 28760 Madrid (ES); JIMENO, J.,Pharma Mar,S.A.U.,Polig.Industr.La Mina, 28770 Madrid (ES)
(74) Representative: Ruffles, Graham Keith
(86) International application number: PCT/GB2002/004816
(87) International publication number: WO 2003/033013

(56) References cited:
- WO-A-01/35974
- WO-A-91/04985
- RAYMOND E ET AL: "PRELIMINARY RESULTS OF A PHASE I AND PHARMACOKINETIC STUDY OF APLIDINE GIVEN AS A 24-HOUR INFUSION EVERY 2 WEEKS IN PATIENTS WITHSOLID TUMORS AND NON HODGKIN'S LYMPHOMAS" PROCEEDINGS OF THE 91ST ANNUAL MEETING OF THE AMERICAN ASSOCIATION FOR CANCER RESEARCH. SAN FRANCISCO, CA, APRIL 1 - 5, 2000, PROCEEDINGS OF THE ANNUAL MEETING OF THE AMERICAN ASSOCIATION FOR CANCER RESEARCH, PHILADELPHIA, PA: AACR, US, vol. 41, March 2000 (2000-03), page 611 XP001002420
- FAIRCLOTH G ET AL: "APLIDINE (APL) IS A NOVEL MARINE-DERIVED DEPSIPEPTIDE WITH IN VIVO ANTITUMOR ACTIVITY" PROCEEDINGS OF THE 89TH. ANNUAL MEETING OF THE AMERICAN ASSOCIATION FOR CANCER RESEARCH. NEW ORLEANS, LA, MARCH 28 - APRIL 1, 1998, PROCEEDINGS OF THE ANNUAL MEETING OF THE AMERICAN ASSOCIATION FOR CANCER RESEARCH, PHILADELPHIA, PA: AACR, US, vol. 39, March 1998 (1998-03), page 227 XP001002422
- FAIRCLOTH G ET AL: "SCHEDULE-DEPENDENCY OF APLIDINE, A MARINE DEPSIPEPTIDE WITH ANTITUMOR ACTIVITY" PROCEEDINGS OF THE 90TH ANNUAL MEETING OF THE AMERICAN ASSOCIATION FOR CANCER RESEARCH. PHILADELPHIA, PA, APRIL 10 - 14, 1999, PROCEEDINGS OF THE ANNUAL MEETING OF THE AMERICAN ASSOCIATION FOR CANCER RESEARCH, PHILADELPHIA, PA: AACR, US, vol. 40, March 1999 (1999-03), pages 394-395, XP001002421

## Description

### TECHNICAL FIELD OF THE INVENTION

The present invention relates generally to therapy of cancer. The invention is more specifically related to the use of aplidine in the manufacture of a medicament for treatment of pancreatic cancer. To prevent the risk of developing tumors, to promote tumor regression, to stop tumor growth, and/or to prevent metastasis

### BACKGROUND OF THE INVENTION

Cancer is a significant health problem throughout the world. Although advances have been made in detection and therapy of cancer, no vaccine or other universally successful method for prevention and/or treatment is currently available.

Current therapies, which are generally based on a combination of chemotherapy or surgery and radiation, continue to prove inadequate in many patients.

Located in the upper abdomen in the retroperitoneum, the pancreas is associated intimately with many major structures including the portal vein, stomach, duodenum, common bile duct and the superior mesenteric artery.

Pancreatic cancer is the fifth leading cause of cancer death in the United States. It is more common among men, and men between the ages of 60 and 70 are most at risk. As the tumor grows, the patient's symptoms result from tumor infiltration of surrounding structure causing pain, nausea, vomiting, weight loss and jaundice. The latter condition presents symptoms in no more than one half of the patients. Once tumour infiltration occurs other structures such as the portal vein become affected and this precludes curative resectioning of the pancreas.

Effective treatment of pancreas cancer must achieve two difficult goals: control of the primary tumour mass, both initially and subsequently, and treatment of the metastatic tumour cells. As a result of its insidious onset, the diagnosis of pancreas cancer is delayed frequently for several months. This delay has profound implications, since metastatic spread to the liver or lymph nodes has been observed at a time of diagnosis in 60% of patients, and this factor diminishes the prospect for long-term survival. Also, the carcinoma of the pancreas is asymptomatic in its early stage. The most common symptoms at later stage are weight loss, abdominal pain, and jaundice. Weight loss, the causes of which are not fully understood, usually is significant. Jaundice occurs if the cancer blocks the common bile duct. By the time the malignant tumour is identified, it often has spread (metastasized) to other parts of the body. The median survival is little more than six months from the time of diagnosis.

Current therapies for this common and difficult-to-treat disease include surgery and/or chemotherapy. Often the tumour cannot be removed by surgery, either because it has invaded vital structures that cannot be removed or because it has spread to distant sites.

Although 5-year survival rates after surgical removal of the pancreas and a large portion of the duodenum have improved, the procedure is only used on 9% of patients. Of these, the highest reported 5-year survival rate is in the range of 20%.

Patients with advanced pancreatic cancer are treated primarily by chemotherapy. The objective of such therapy is to prolong patient survival. Surgery and irradiation are used as well to relieve pain and reduce organ blockage.

In spite of considerable research into therapies for these and other cancers, pancreatic cancer remains difficult to treat effectively.

Accordingly, there is a need in the art for improved methods for treating primary and metastatic pancreatic cancers. The present invention fulfills these needs and further provides other related advantages.

Dehydrodidemnin B, now known as aplidine, is the subject of W091/04985.

Further information on aplidine is to be found in, for example : Jimeno, J.,"Exploitation of marine microorganisms and invertebrates : Anticancer drugs from marine origin", IBC Conf Discov Drugs from Nat Novel Approaches New Sources (Dec 8-9, London) 1994, 1994; Faircloth, G. et al.,"Dehydrodidemnin B (DDM) a new marine derived anticancer agent (MDA) with activity against experimental tumour models", 9th NCI-EORTC Symp New Drugs Cancer Ther (March 12-15, Amsterdam) 1996, Abst 111; Sakai, R. et al.,"Structure-activity relationships of the didemnins", Journal of Medicinal Chemistry 1996, 39 (14) : 2819; Urdiales, J. L. et al. "Antiproliferative effect of dehydrodidemnin B (DDB), a depsipeptide isolated from Mediterranean tunicates", Cancer Letters 1996, 102 (1-2) : 31; Faircloth, G. et al.,"Preclinical characterization of aplidine (APD), a new marine anticancer depsipeptide (MADEP)", Proc Amer Assoc Cancer Res. 1997, 38 : Abst 692; Depenbrock, H. et al. "In vitro activity of aplidine, a new marine-derived anti-cancer compound, on freshly explanted clonogenic human tumour cells and haematopoietic precursor cells", British Journal of Cancer 1998, 78 (6) : 739; Faircloth, G. et al.,"Aplidine (aplidine) is a novel marine-derived depsipeptide with in vivo antitumour activity", Proc Amer Assoc Cancer Res 1998, 39 : Abst 1551; Faircloth, G. et al.,"Preclinical development of aplidine, a novel marine derived agent with potent antitumour activity", 10th NCI-EORTC Symp. New Drugs Cancer Ther (June 16-19, Amsterdam) 1998, Abst 129; Geldof AA, Mastbergen SC, Henrar RE, Faircloth GT. "Cytotoxicity and neurocytotoxicity of new marine anticancer agents evaluated using in vitro assays", Cancer Chemother Pharmacol 1999;44(4):312-8; Jimeno J, Smith B, Grant W, Faircloth GT. "A correlation of selective antitumour activities of the marine-derived compound aplidine using different models" 10th NCI-EORTC-AACR Symposium on molecular targets and cancer therapeutics. Washington, 1999. Abstract 311; Broggini M. Marchini S, D'Incalci M, Taraboletti G, Giavazzi R, Faircloth G, Jimeno J. "Aplidine blocks VEGF secretion and VEGF/VEGF-RI autocrine loop in a human leukemic cell line", 11th NCI-EORTC-AACR Symposium on new drugs in cancer therapy. Amsterdam, 2000. Abstract 21; Luber-Narod, J., Jimeno, J.; Faircloth, GT. et al. "In vitro safety profile of aplidine, a marine natural product with chemotherapeutic potential", Proceedings of the AACR, vol. 42, abstract 374, March 2001.

In preclinical studies, aplidine had dose-dependent cytotoxic activity against the two epithelial-like cell lines, CT-1 and CT-2, and the human colon cancer cell line, HT-29. The most proliferative line, CT-2, was the most sensitive to aplidine. In addition the compound decreased ornithine decarboxylase activity in all three cell lines (Lobo C, Garcia Pozo SG, et al.: "Effect of dehydrodidemnin B on human colon carcinoma cell lines", Anticancer Research. 17 : 333-336, Jan-Feb 1997). In a similar study, aplidine 50 nmol/L inhibited the growth of the breast cancer cell lines, MDA-MB231 and MCF-7 by 17 and 47%, respectively.

Continuous exposure to low concentrations of aplidine inhibited the growth of a number of tumour cell lines, including non-Hodgkin's lymphoma, melanoma, breast, ovarian and non-small cell lung cancers. The magnitude of effect was dependent on the time of exposure and appeared to be achievable at non-myelotoxic concentrations. Non-small cell lung cancer, breast cancer and melanoma cell lines were sensitive to a continuous exposure to aplidine at concentrations of > = 0.001 micromol/L. aplidine had similar toxicity to doxorubicin against clonogenic haematopoietic stem cells (Depenbrock H, Peter R, et al.: "In vitro activity of aplidine, a new marine-derived anti-cancer compound, on freshly explanted clonogenic human tumour cells and haematopoietic precursor cells", British Journal of Cancer. 78 : 739-744, No. 6, Sep 1998).

In general drug therapies are evaluated with respect to treating human cancer, e.g., human cancer xenograft lines. Human tumours are serially heterotransplanted into immunodeficient, so-called "nude" mice, and the mice then tested for their responsiveness to a specific drug. (Giovanella, B. C., et al., Cancer 52(7):1146 (1983)). The data obtained in these studies strongly support the validity of heterotransplanted human tumours into immunodeficient mammals, such as nude mice, as a predictive model for testing the effectiveness of anticancer agents.

Aplidine had significant activity against mice bearing human cancer xenografts. At a maximum tolerated dose of 2.1 mg/kg, aplidine produced near complete remissions in some animals with a treated/control (T/C) tumour ratio of 9%. At 1.25 mg/kg, significant activity was seen against gastric tumours (T/C 14%) and prostate tumour growth inhibition was also observed (T/C 25%) (Faircloth G, Grant W, et al. "Preclinical development of aplidine, a novel marine derived agent with potent antitumour activity", Annals of Oncology. 9 (Suppl. 2) : 34, 1998). aplidine was also active against P388 leukaemia and B 16 melanoma implanted in mice, with an optimal dose of 160 micro/kg. Unlike didemnin B, aplidine was active in SC implanted lewis lung carcinomas (F'aircloth 0, Rinehart K, et al.: "Dehydrodidemnin B a new marine derived anticancer agent with activity against experimental tumor models", Annals of Oncology. 7 (Suppl. 1): 34, 1996).

The use of aplidine for the treatment of different cancers is described in WO 0135974 of the present applicant. Examples 17 and 18 of this application state that aplidine was tested in phase I clinical trials to determine toxicity (Maximum Tolerated Dose) and pharmacokinetic data. Some of the patients had pancreatic tumor, although no hint of activity for these tumors was reported. It is the case that these 4 patients did not respond to the aplidine that was administered to them.

### SUMMARY OF THE INVENTION

We have for the first time established that apildine induces an antitumour effect in primary tumors and metastases in human pancreatic carcinoma models, in a tumor specific manner.

Thus, the present invention provides the use of aplidine in the manufacture of a medicament for the treatment of any mammal, in particular a human, affected by pancreatic cancer to prevent the risk of developing tumors, to promote tumor regression, to stop tumor growth, and/or to prevent metastasis.

The present invention provides the use of aplidine in the manufacture of a medicament for the treatment of any mammal, in particular a human, affected by metastatic pancreatic cancer to prevent the risk of developing tumors, to promote tumor regression, to stop tumor growth, and/or to prevent metastasis.

### DESCRIPTION OF THE FIGURES

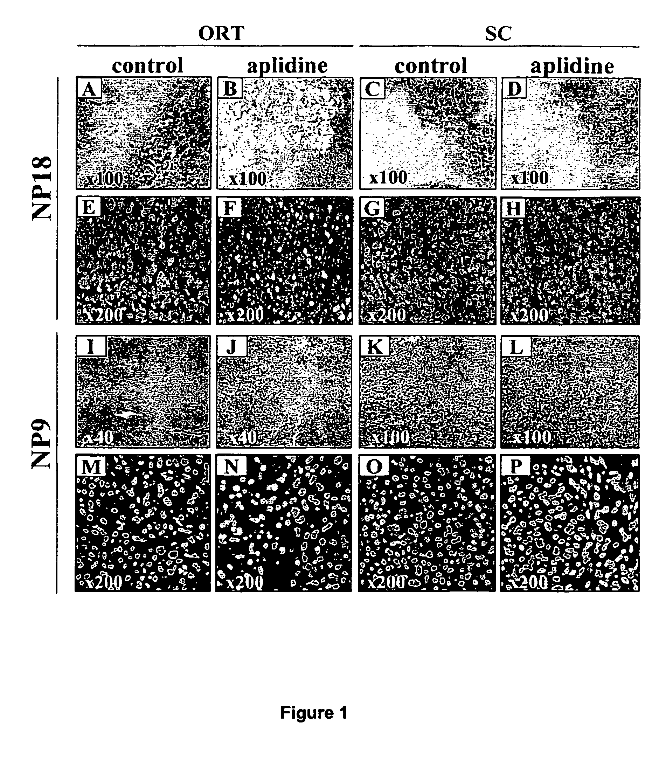

**Figure 1****.** Aplidine induces necrotic and apoptotic cell death in the NP18 (panels A-H) and NP9 (panels I-P) tumours only at the ORT site: H&E staining of ORT NP18 aplidine-treated tumours showed scar fiber replacement of necrotic tissue (panel B), which was not observed in the ORT control group (panel A), nor in treated (panel D) or control (panel C) SC tumours. Hoescht staining of viable ORT NP18 treated tumours depicted nuclear condensation (panel F). No induction of nuclear condensation was observed in control ORT (panel E), SC treated (panel G) or SC control (panel H) tumours. In the ORT NP9 treated tumour, we observed multiple foci of necrosis occupying most of the tumour (panel J). These foci were significantly smaller or not observed in the control ORT (panel I), nor in the treated (panel L) or control (panel K) SC groups. Nuclear condensation was only observed in the viable ORT NP9 treated tumours (panel N), but it was not in control ORT (panel M), SC treated (panel O) or SC control (panel P) tumours.

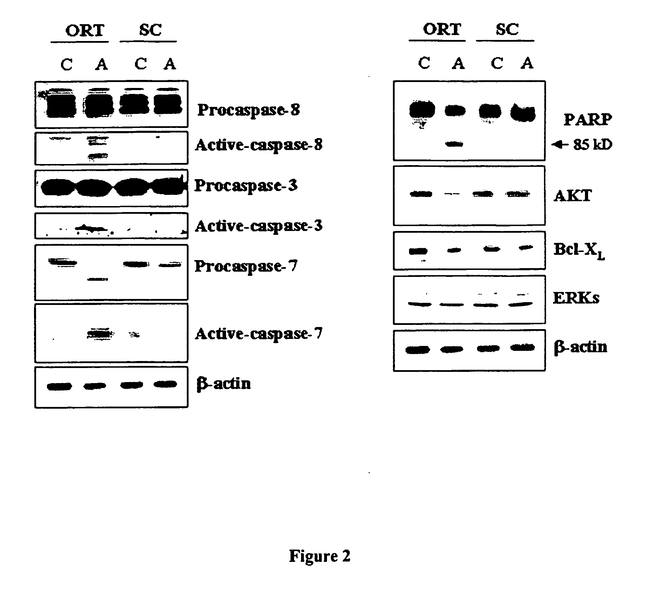

**Figure 2****.** Aplidine activates apoptotic pathways in the NP18 tumour: Decrease AKT activation, activation of caspases 3, 7 and 8 and PARP cleavage were detected in ORT aplidine-treated (T), as compared to control (C) tumours. At the SC site, aplidine did not alter the regulation of any of these proteins. A similar decrease of Bcl-X_{L} protein was detected in ORT or SC aplidine-treated tumours, as compared with their respective vehicle controls. No differences in ERK activation were observed between treated and control tumours in any site.

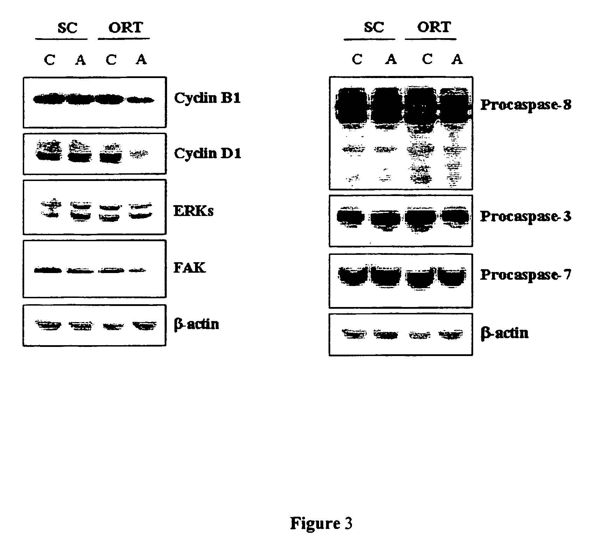

**Figure 3****.** Aplidine downregulates cell cycle regulatory molecules in the NP9 tumour: A decreased expression of cyclin B1 and cyclin D1 was detected in ORT aplidine-treated (T) as compared with ORT control (C) tumours. In contrast, no differences in cyclin B1 or cyclin D1 levels were observed between treated and control SC tumours. No active forms of caspases 3, 7 or 8 were detected, despite of detecting their respective procaspases in NP9 control (C) or treated (T) tumours; however, downregulation of FAK was observed only in the ORT-treated tumours. ERK activation was unaltered by treatment in both tumour site. Expression of beta-actin was used to control for equal protein loading.

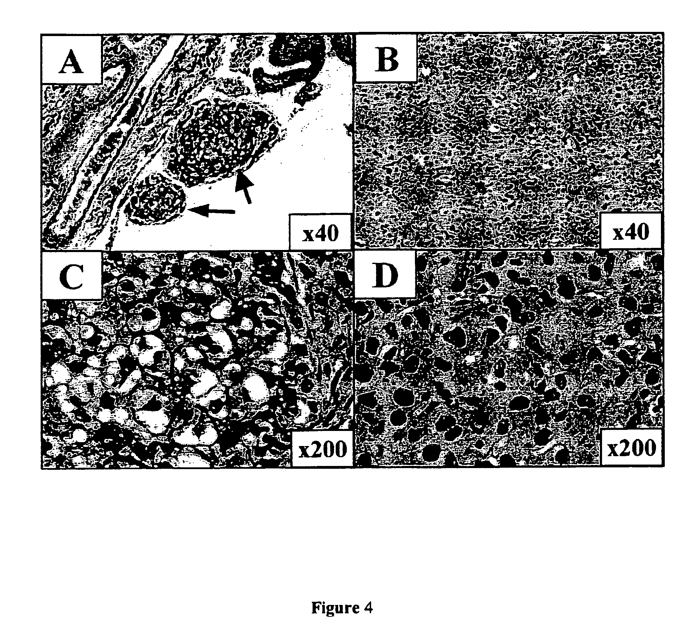

**Figure 4****.** H&E staining of aplidine-treated NP9 peritoneal metastasis: aplidine-treated implants (Panel A (40X magnification) and B(200X magnification)) did not contain necrotic areas. No differences in necrosis were observed in aplidine-treated implants, as compared to control implants (panel C (X200) and D (X200)). A high percentage of tumour cells with big and white cytoplasm were detected in aplidine-treated (panel B) as compared with control (panel D) implants.

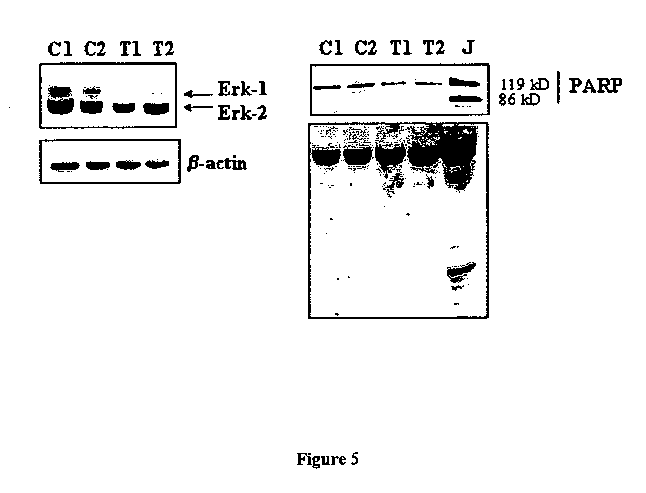

**Figure 5****.** Molecular analysis of aplidine-treated NP9 peritoneal metastasis: A decreased in ERKs activation was detected only in treated (T) NP9 peritoneal metastasis. No activation of caspase-3 nor PARP proteolysis were detected in control (C.) or treated (T) implants. J, Control apoptotic DNA from Jurkat cells treated with camptothecine.

### DETAILED DESCRIPTION OF THE INVENTION

Pancreatic cancer *in vivo* models were tested with aplidine to find evidence of activity. Surprisingly, we found out that aplidine induces necrosis, apoptosis and cell cycle arrest in two primary human pancreatic carcinomas (NP9 and NP18) xenotransplanted in nude mice and very specially inhibits the growth of the peritoneal metastases in the tested NP9 tumour.

Therefore aplidine, or a pharmaceutical composition thereof, will be effective in the treatment of pancreatic cancer.

Aplidine has the following formula:

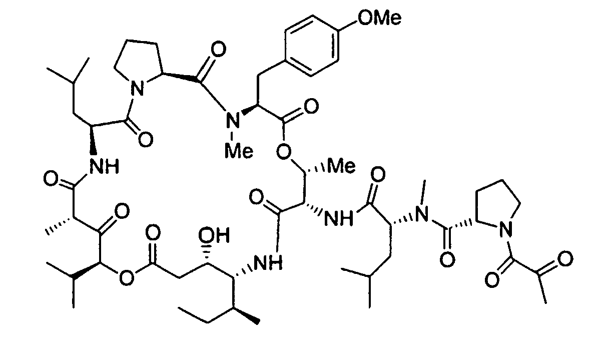

Examples of pharmaceutical compositions containing aplidine include liquid (solutions, suspensions or emulsions) with suitable composition for intravenous administration, and they may contain the pure compound or in combination with any carrier or other pharmacologically active compounds.

Solubilised aplidine shows substantial degradation under heat and light stress testing conditions, and a lyophilised dosage form was developed, see W099/42125 incorporated herein by reference. In a currently preferred embodiment freeze-drying was performed from a 500 mg/mL solution of aplidine in 40% (v/v) tert-butanol in Water for Injection (Wf1) containing 25 mg/mL D-mannitol as bulking agent. The prototype, containing 500 mg aplidine and 25 mg D-mannitol as bulking agent per vial was found to be the optimal formulation in terms of solubility, length of lyophilisation cycle and dosage requirements in the clinical studies. The optimal reconstitution solution was found to be 15/15/70% (v/v/v) Cremaphor EL/ethanol/Wfi (CEW). Both reconstituted product and dilutions (up to 1: 100 v/v) of the reconstituted product with normal saline appeared to be stable for at least 24 hours after preparation. Shelf-life data, available thus far, show that the formulation is stable for at least 1 year when stored at 4 C in the dark.

Administration of aplidine or compositions as described herein is based on a Dosing Protocol preferably by intravenous infusion. We prefer that infusion times of up to 72 hours are used, more preferably 1 to 24 hours, with about 1, about 3 or about 24 hours most preferred. Short infusion times which allow treatment to be carried out without an overnight stay in hospital are especially desirable. However, infusion may be around 24 hours or even longer if required. Infusion may be carried out at suitable intervals with varying patterns, illustratively once a week, twice a week, or more frequently per week, repeated each week optionally with gaps of typically one week.

Depending on the type of tumor and the developmental stage of the disease, the uses of the invention are useful in preventing the risk of developing tumors, in promoting tumor regression, in stopping tumor growth and/or in preventing metastasis.

The correct dosage of the compound will vary according to the particular formulation, the mode of application, and the particular situs, host and tumor being treated. Other factors like age, body weight, sex, diet, time of administration, rate of excretion, condition of the host, drug combinations, reaction sensitivities and severity of the disease shall be taken into account. Administration can be carried out continuously or periodically within the maximum tolerated dose. Further guidance is given in WO 0135974.

The compound aplidine and compositions as described herein may be used with other drugs to provide a combination therapy. The other drugs may form part of the same composition, or be provided as a separate composition for administration at the same time or a different time. One preferred drug to be combined with aplidine is carnitine.
Also preferably, aplidine is administered in conjunction with one or more chemotherapeutic agents effective against pancreatic cancer such as gemcitabine or 5-FU.

According to the present invention, we demonstrated that aplidine induces necrosis, apoptosis and cell cycle arrest in two primary human pancreatic carcinomas (NP9 and NP 18) xenotransplanted in nude mice and inhibits the growth of the peritoneal metastases in the tested NP9 tumor.

Aplidine has antitumour activity against the NP18 tumor, when implanted at the ORT site, through apoptotic and necrotic induction, followed by scar fiber formation. Apoptosis appears to be mediated by the AKT pathway, since we observed inhibition of AKT activation and caspases 3, 7 and 8 activation leading to PARP cleavage in these tumors. Other growth regulatory pathways, such as the ERKs remained unchanged. Diminished AKT activation is a common finding in drug-induced apoptosis.

Very significantly, aplidine has antitumour activity against the NP9 tumor, wen implanted at the ORT site, and its mechanism of action appear to be a combination of induction of growth arrest and cell death. This is based on the observed microscopic necrosis, and on the downregulation of cyclins DI and B 1 and Ki67 and high percentage of nuclear condensation, by Hoescht staining, in the viable tissue in these tumors. Thus, in spite of not observing a reduction in tumor size, this was likely to occur at a later time, as a consequence of the resorption of these necrotic foci. Therefore, aplidine induces a tumour-specific alteration of apoptotic and/or proliferative pathways which could explain its antitumour effect when implanted at the ORT site.

In addition to the antitumour effect of aplidine against the primary pancreatic carcinomas, we show here that this compound is also antimetastatic in the NP9 tumour. This antimetastic effect was demonstrated, both, when treatment started before metastatic implantation and by the growth inhibition of previously established metastatic implants.

The metastatic foci that had 1-2 mm before treatment, disappeared completely or had undergone a significant reduction in size and became only detectable under the microscope, showing no necrotic areas. Therefore, the most likely mechanism for their reduction in size was, not necrosis, but apoptotic induction.

Interestingly the molecular response of metastases and primary tumour to the drug differed (ERK activation was decreased in metastases and unchanged in the primary tumour); implying that the response of the metastases could not be anticipated from that observed in the primary tumour. Our observation is very relevant because, to date, no effective therapy has been described for the treatment of metastatic pancreatic carcinoma. Based on these findings a phase II clinical trial on exocrine pancreatic cancer was designed.

### EXAMPLES

### Preliminary Report

### Growth inhibitory and antimetastatic activity of aplidine in human pancreatic carcinoma xenografts in nude mice

Aplidine is a new cyclic depsipeptide with anticancer activity in vitro and in vivo in different tumour types, but it has not been tested in pancreatic carcinoma. Nevertheless, this pathology has a poor prognosis because of difficult early diagnosis, aggressiveness and lack of effective systemic therapies. Most patients die of metastases present at diagnosis. We compared the antitumour effect of aplidine against 2 human pancreatic carcinomas, NP9 and NP18, using orthotopic (ORP) and subcutaneous (SC) tumour implantation models. NP9 tumour shows an extensive peritoneal dissemination 3-4 weeks after implantation. METHODS: Groups of ten male Nu/Nu Swiss mice (4 wk old) were treated with vehicle or aplidine. To evaluate primary tumour response, ip aplidine was given, starting 2 weeks (wk) after implantation, at .8 mg/kg q4d for 4 wk (NP9) or at .8 mg/kg q4d for 6 wk, ceasing treatment the 4th and 5th wk (NP18). To evaluate the response of NP9 metastasis aplidine regime was .8 mg/kg q4d per 3 doses, starting 4 weeks after implantation. We measured final tumour weight, macroscopic viability (viable cortex/total tumour weight), microscopic necrosis, apoptotic induction and signal transduction. RESULTS: aplidine showed antitumour effect on NP9 and NP18 primary tumours, which was exclusively observed at the microscopic and molecular level on the ORT site, not at the SC site, in both tumours. This effect was due to apoptotic induction leading to a reduced number of viable cells in both tumours. Apoptotic induction on NP18 was associated with PARP cleavage and reduced AKT activation, only in ORT-treated tumours. aplidine induced a decrease in cyclins B1 and D1 and FAK expression only in ORT NP9-treated tumours. In addition, aplidine showed complete inhibition of NP9 peritoneal implants, whether the treatment started before or after the occurrence of the metastatic deposits. Absence of necrosis and a decrease in MAPK activity were observed in treated metastases. CONCLUSIONS: The use of an ORT implantation model permitted the observation of the antitumour effect of aplidine on primary tumours and metastases of human pancreatic carcinomas. aplidine altered apoptotic and proliferative pathways, which could explain its antitumour effect. Moreover, ORT and SC implanted tumours showed a different response to this drug.

### Detailed Report

We used two human pancreatic carcinomas (NP18 and NP9) perpetuated as orthotopic xenografts in nude mice. NP9 produce an extensive peritoneal dissemination 4-5 weeks after implantation (metastatic). We implanted these tumours in four week-old male Nu/Nu Swiss mice (Charles River), housed in autoclaved cages and provided γ-ray sterilized bedding and food. They were anesthetized with 2,2,2-tribromoethanol (Sigma-Aldrich) and 10 mg fragments of each tumour from previous passages were implanted orthotopically (ORT, at the pancreas). Xenografted animals were randomly distributed in control (n=10) and experimental (n=10) groups just before treatment. We excluded animals bearing non palpable tumours after ORT implantation, or with tumour volumes lower than 0.13 cm³ (0.5 cm in diameter). Treatment schedule depended on tumour growth rate. In the fast growing NP9 tumour, two schedules of treatment were used. In one, treatment started two weeks after tumour implantation, before peritoneal dissemination had occurred. Each animal in the experimental group received ip administrations of aplidine at a dose of 0.8 mg/Kg q4d per four weeks. In the other schedule, treatment started four weeks after tumour implantation, once peritoneal dissemination had occurred, with a regime of aplidine of 0.8 mg/Kg q4d per three doses. The existence of macroscopically visible implants was confirmed one day before the treatment begun, by the sacrifice of one extra animal. In the NP18 tumour, treatment started 4 weeks after tumour implantation and the schedule of aplidine was 0.8 mg/Kg q4d ip for 4 weeks, ceasing treatment for two weeks, and continuing with 0.8 mg/Kg q4d ip for two additional weeks. The animals in the corresponding control groups received i.p. administrations of vehicle following the same schedule. After implantation, and during treatment, mice were inspected twice a week and weighted weekly to monitor for compound toxicity.

At the end of treatment, animals were euthanized and dissemination recorded. Primary tumours were excised and weighted in all groups. Tumor aliquots were frozen down in liquid nitrogen or fixed in formaline. We counted the number of peritoneal implants in each animal and took samples for histological and molecular analysis. The mean of the total and solid (central necrosis excluded) tumour weight was compared between groups, using the Student t test, the differences being significant at p<.05.

### Histological analysis and Hoescht test

For histopathological analysis, formalin-fixed paraffin-embedded tissues were stained with H&E following the standard protocol and the presence or absence of microscopic cell death were observed. Nuclear staining of DNA was performed in 5 □m sections of formalin-fixed paraffin-embedded tumour tissue. Sections were dewaxed in xylene and dehydrated, washed twice with PBS, permeabilized with triton X-100 for 10 min at RT and washed twice with PBS. Then, the slides were incubated with Hoescht (1:5.000 in PBS) for 1h, washed with sterile water and dried at RT. Finally, the slides were mounted and observed under a flourescence microscope, analyzing only areas not involving microscopic necrosis.

### Western Blot analysis

Molecular analyses were performed in whole cell protein extracts of tumours by Western blot, as described (11). The anti-active MAPK Ab (Promega) (1:20,000) detected active Erk-1 and Erk-2 forms. The rabbit anti-cyclin B1 (1:1,000), anti-Bcl-XL (1:7,000-o/n), and anti-FAK (diluted 1:7.000) and the goat anti-β-actin (1:15,000) polyclonal Abs were from Santa Cruz. The rabbit anti-Cyclin D1 was from Upstate Biotech. The rabbit anti-caspase-3, anti-caspase-7 and anti-caspase-8 Abs were from Pharmingen, the rabbit anti-active-AKT from New England Biolabs, and the rabbit anti-PARP from Boehringer-Mannheim. All secondary antibodies (Jackson ImmunoResearch) were used at a dilution of 1:10,000.

### Immunohistochemistry analysis

IHC analysis was performed on paraffin-embeded tumour tissue. Five □m sections were dewaxed in xylene and dehydrated. Endogenous peroxidase activity was blocked with 0.03% H₂O₂. Antigen was retrieved heating and incubating with 10mM citric acid monophosphate buffer (pH 6.0). Sections were incubated with anti-Ki67 Ab at RT for 1 h and, then, with secondary Ab peroxidase conjugated EnVision TM (Dako) for 30 min at RT, followed by DAB+ cromogen (Dako) and counterstaining with hematoxyline. Sample quantitation was done counting positively stained cells in three random high power (100X) fields. Differences between groups were considered significant at p<.05 (Student t test).

### RESULTS

We first analyzed the effect of aplidine on NP18 and NP9 human pancreatic primary tumours after aplidine treatment. We describe the macroscopic and microscopic changes, and the alteration of the expression and/or activation of apoptotic and cell cycle regulatory proteins. We also describe the effect of aplidine on the metastatic implants at the macroscopic, microscopic and molecular level.

### Effect of aplidine on the primary tumours

Aplidine altered the macroscopic appearance of the NP9 primary tumour at the ORT site. Tumor margins were very well defined in the ORT treated group, since they did not invade other organs of the peritoneum, making its dissection easy. In contrast, tumours in ORT control animals invaded the spleen and the wall of the peritoneum. After aplidine treatment the size or macroscopic appearance of the NP18 primary tumour did not significantly change, as compared with its control.

### Effect on tumour weight

Once primary tumours were excised we analyzed the effect of aplidine on tumour weight. At the end of the treatment period, there were no significant differences in total or solid tumour weight between the aplidine-treated and the control group in both tumours at any site. Final tumour weights are described in Table 1.

| | | | **Total tumour weight (g)** | **Solid tumour weight (g)** |
|---|---|---|---|---|
| **NP9** | **SC** | Control | 2.46±0.6^{a} | 1.04±0.33^{e} |
| | | treated | 1.56±0.8^{a} | 0.87±0.44^{e} |
| | **ORT** | control | 2.74±0.85^{b} | 2.15±0.51^{f} |
| | | treated | 3.72±1.09^{b} | 3.04±1.04^{f} |
| **NP18** | **SC** | control | 17.7±5.9^{c} | - |
| | | treated | 15.5±5.3^{c} | - |
| | **ORT** | control | 9.96±3.5^{d} | - |
| | | treated | 9.34±1.3^{d} | - |

| | | | | |
|---|---|---|---|---|
| ^{a,b,c,d,e,f} No significant differences (Student T test) | | | | |

### Histopathological analysis

In the NP18 model, we observed a significantly higher level of necrosis in the ORT treated than in ORT control tumours. Moreover, scar fibers filled the space left by extensive areas of dead tumour cells, exclusively in the ORT treated tumours. In the NP9 tumour, the foci of non-viable/necrotic cells occupied 75-100% of each tumour. In contrast, necrosis occupied only 0-25% of each tumour in control NP9 ORT tumours. Therefore, aplidine significantly increased the areas of microscopic necrosis in both the NP9 and NP18 primary tumours.

### Hoescht nuclear staining

The analysis of the histologically viable tissue, in both NP18 and NP9 tumours, after nuclear staining with Hoescht dye, revealed enhanced density of picnotic nuclei with condensed and fragmented chromatin in the ORT treated tumours, which were not observed in the ORT control tumours. Therefore, aplidine significantly increased apoptosis in both the NP18 and NP9 tumours.

### Effect of aplidine on the NP9 peritoneal implants

We tested the possible antimetastatic effect of aplidine on the ORT NP9 tumour since it produced early and massive peritoneal dissemination. In the ORT control group, we recorded more than 100 implants (1-2 mm diameter) per animal, four weeks after implantation.

After completion of aplidine treatment, which initiated before microscopically visible dissemination had occurred, no peritoneal implants were observed in any animal. In addition, aplidine also inhibited the growth of the metastases when treatment started once they were macroscopically evident, since animals in the treated group had only a few (7-20) and small implants (less than 1 mm diameter), whereas, control animals presented more than 50 implants, reaching 3-4 mm of diameter. Therefore, aplidine completely abolishes peritoneal dissemination of the ORT implanted NP9 tumour, and it is able to inhibit the growth of already established metastatic implants.

### Pathological and molecular analysis of NP9-treated metastasis

We histopathologically analyzed the macroscopically visible implants in aplidine-treated and vehicle treated animals. Despite the significantly smaller size of the metastatic foci in experimental than in control animals, aplidine-treated metastases did not show higher cell death than control metastases, at the time of the analysis, since tumour tissue in both group was microscopically viable. Nuclear staining with Hoescht showed a few (3-6) apoptotic nuclei per treated implant, but no significant differences were observed between aplidine-treated and control metastases in number of apoptotic nuclei. Molecular analysis revealed that NP9 aplidine-treated had lower ERK activation than control metastases. No activation of Caspase-3 nor PARP cleavage were observed in treated metastasis. Thus, the inhibition of growth in metastases associated with a reduction in ERK activation.

## Claims

1. The use of aplidine in the preparation of a medicament for the treatment of
a mammal affected by pancreatic cancer to prevent the risk of developing tumors, to promote tumor regression, to stop tumor growth, and/or to prevent metastasis.

2. The use according to claim 1, wherein the pancreatic cancer is metastatic pancreatic cancer.

3. The use according to claim 1 or 2, wherein the mammal affected by pancreatic cancer is human.

4. The use according to any preceding claim, wherein the pancreatic cancer is refractory to other treatments.

5. The use according to any preceding claim, wherein the aplidine is administered simultaneously or sequentially in combination with another drug.

6. The use according to claim 5, wherein the other drug is a chemotherapeutic agent against cancer.

7. Aplidine for use in the treatment of a mammal affected by pancreatic cancer to prevent the risk of developing tumors, to promote tumor regression, to stop tumor growth, and/or prevent metastasis.

## Patentansprüche

1. Anwendung von Aplidin bei der Herstellung eines Arzneimittels zur Behandlung eines Saugers, der von Bauchspeicheldrüsenkrebs betroffen ist, um dem Risiko Tumore zu entwickeln vorzubeugen, die Tumor-Regression zu begünstigten, das Tumorwachstum aufzuhalten, und/oder Metastasen vorzubeugen.

2. Anwendung nach Anspruch 1, wobei der Bauchspeicheldrüsenkrebs ein metastatischer Bauchspeicheldrüaenkrebs ist.

3. Anwendung nach Anspruch 1 oder 2, wobei der von Bauchspeicheldrüsenkrebs betroffene Säuger ein Mensch ist.

4. Anwendung nach irgendeinem vorhergehenden Anspruch, wobei der Bauchspeicheldrüsenkrebs auf andere Behandlungen nicht anspricht.

5. Anwendung nach irgendeinem vorhergehenden Anspruch, wobei Aplidin simultan oder sequentiell in Verbindung mit einem anderen Arzneimittel verabreicht wird.

6. Anwendung nach Anspruch 5, wobei das andere Arzneimittel ein chemotherapeutisches Mittel gegen Krebs ist.

7. Aplidin zur Anwendung bei der Behandlung eines Säugers, der von Bauchspeichledrüsenkrebs betroffen ist, um dem Risiko Tumore zu entwickeln vorzubeugen, die Tumor-Regression zu begünstigen, das Tumorwachstum aufzuhalten, und/oder Metastasen vorzubeugen.

## Revendications

1. Utilisation d'aplidine dans la préparation d'un médicament pour le traitement d'un mammifère atteint d'un cancer du pancréas pour empêcher le risque de développement de tumeurs, pour promouvoir la régression de la tumeur, stopper sa croissance, et/ou empêcher les métastases.

2. Utilisation selon la revendication 1, dans laquelle le cancer du pancréas est un cancer pancréatique métastatique.

3. Utilisation selon la revendication 1 ou 2, dans laquelle le mammifère atteint du cancer du pancréas est un humain.

4. Utilisation selon une quelconque revendication précédente, dans laquelle le cancer du pancréas est réfractaire à d'autres traitements.

5. Utilisation selon une quelconque revendication précédente, dans laquelle l'aplidine est administrée de manière simultanée ou séquentielle en combinaison avec un autre médicament.

6. Utilisation selon la revendication 5, dans laquelle l'autre médicament est un agent chimiothérapeutique contre le cancer.

7. Aplidine pour une utilisation dans le traitement d'un mammifère atteint d'un cancer du pancréas pour empêcher le risque de développement de tumeurs, pour promouvoir la régression de la tumeur, stopper sa croissance, et/ou empêcher les métastases.
